Europäisches Patentamt

European Patent Office

Office européen des brevets

(19)

(11) **EP 1 691 311 A1**

(12) # EUROPEAN PATENT APPLICATION

(43) Date of publication:
**16.08.2006 Bulletin 2006/33**

(51) Int Cl.:
***G06F 19/00*** (2006.01)

(21) Application number: **05290333.3**

(22) Date of filing: **15.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Institut Gustave Roussy**
**94805 Villejuif Cédex (FR)**

(72) Inventors:
• **Kaufmann, Audrey**
**94230 Cachan (FR)**

• **Dessen, Philippe**
**94200 Ivry sur Seine (FR)**
• **Lazar, Vladimir**
**94800 Villejuif (FR)**

(74) Representative: **Hassine, Albert**
**Cabinet Plasseraud**
**65/67 rue de la Victoire**
**75440 Paris Cedex 09 (FR)**

(54) **Method, system and software for carrying out biological interpretations of microarray experiments**

(57)     The invention relates to DNA microarray data analysis. A p-value of each gene of a cluster, such as a pathway given by a database, is calculated on the basis of fluorescence signals issued from a microarray chip, corresponding to a probability that the gene is reported as being differentially expressed between several cell types, under a certain condition. Clusters from pre-existing databases containing a given number of genes of the microarray are formed, and, for each cluster, significant genes are selected with respect to their p-values. According to the invention, genes of the microarray are randomly drawn in order to evaluate a Z-score indicating a significance of each cluster.

EP 1 691 311 A1

**Description**

This invention relates to searching biological interpretations from microarray experiments. Specifically the present invention relates to statistical methods for functional analysis and identification of differentially expressed genes of microarray data.

The present invention also relates to statistical methods for determining the molecular functions of genes and/or the metabolic pathways and/or the interaction networks and/or biological processes involving the gene products and/or the sub-cellular localization of said gene products and/or the chromosomal localization of said genes.

Biological systems are controlled by hundreds of genes working in concert. Microarray technology allows to monitor simultaneously thousands genes and identify a subset of differentially expressed gene in a particular cell type under a given biological or pathological condition. Changes of gene expression levels illustrate modification in gene functions.

The basic concept of microarray analysis is simple. Nucleic acids from a cell type are harvest and labeled to generate targets. Said nucleic acids are hybridized to probe DNA sequences (cDNA or oligonucleotides) corresponding to specific genes that have been affixed onto a solid matrix in a known configuration. Hybridization, based on base-pairing between probes and targets, provides a quantitative measurement of the abundance of a particular gene of the target nucleic acids. This information is captured digitally and subject to analysis. Comparison of hybridization pattern enables the identification of nucleic acids that differs in abundance in two or more target samples.

Simulations are powerful tools for understanding dynamic complexity and envisioning a variety of outcomes through experimentation.

In particular, when microarray experiments are to be analyzed, the genes are filtered on a criteria based, for example, on p-value, fold change, log(ratio) and/or intensity. Then, various statistical tests can be performed to refine the selection so that the found genes can fit correctly. Finally, common properties between these genes defined as having an interest can be found. This last step often consists in clustering genes by molecular functions or biological processes according to the Gene Ontology consortium (Ashbumer *et al.*, Nature Genetics, 2000; 25: 25-29), for instance, FatiGO (Al-Shahrour et *al.*, Bioinformatics, 2004; 20: 578-580), GOminer (Zeeberg *et al.,* Genome Biology, 2003; 4:R28), MAPPFinder (Doniger *et al.,* Genome Biology, 2003; 4:R7) or by pathways from KEGG (Ogata *et al.,* Nucleic Acids Research, 1999; 27: 29-34.) with DAVID (Dennis *et* al., Genome Biology, 2003; 4:P3) or from BioCarta (http://www.biocarta.com/genes/index.asp).

While analyzing the approaches of the prior art, it has been realized that an "a priori" that can either be right or wrong was imposed during the first two steps.

For instance, genes with a low fold change were simply not taken into account, thus ruling out potentially important regulation pathways.

Statistical analysis of microarray data is probably the most difficult problem associated with the use of this technology. The aim is to apply statistical approaches to determine gene expression change significance under a given biological or pathological condition, thus enabling the extraction of significant biological information.

It is therefore an object of the present invention to provide statistical methods for identifying significant changes of gene expression, to cluster genes, and to determine whether the differences among individual genes and/or clusters of genes are significant in identifying differentially expressed genes in different cell types, under different biological or pathological conditions.

The present invention aims a method as claimed in claim 1. Embodiments of the method are aimed in dependent claims. The invention aims also a system for performing the method. The invention aims also a computer program product for implementing all or some of the steps of the method.

Other advantages and characteristics of the invention appear on reading the following detailed description of an implementation given below by way of example with reference to the accompanying figures, in which:

- Figure 1A represents a microarray fluorescence readout;
- Figure 1 B shows an outline of the algorithm of the present invention;
- Figure 2a shows an expression data set of a microarray experiment wherein for each sample of interest, each gene is associated with a measurement of fluorescence intensity generated from the microarray fluorescence readout as shown in Figure 1A;
- Figure 2b shows a table wherein each gene is assigned to a p-value;
- Figure 2c shows a table wherein genes are clustered into pathways according to information retrieved from a pre-existing database;
- Figure 2d shows a table wherein each pathway of Figure 2c is assigned to p-values (listed in the table of figure 2b) associated to its constituting genes;
- Figure 2e shows a table wherein each pathway is assigned to a percentage of significant genes;
- Figure 2f shows a table wherein each pathway is assigned to randomized p-values;
- Figure 2g shows a table wherein each pathway is assigned to a percentage of significant genes found randomly;
- Figure 2h shows a table wherein each pathway is characterized by a Z-score value;
- Figure 3 shows an example of representative results table consisting of a rank list of clusters generated by the implementation of the present invention;
- Figure 4a shows an expression data set of a microarray experiment as shown in Figure 2a;

- Figure 4b shows a table wherein each pair of genes is assigned to a correlation estimator;
- Figure 4c shows a table wherein known and unknown genes are grouped with respect to the correlation estimators;
- Figure 4d shows a table wherein known genes are clustered into pathways which are characterized by Z-score values;
- Figure 4e shows a table wherein unknown genes are assigned to clusters of known genes;
- Figure 5 shows a system for performing the invention;
- Figure 6 shows a representative flowchart of an embodiment of a main algorithm of the software according to the invention;
- Figure 7 shows a flowchart algorithm for calculating a Z-score value (z-score); assigned to a cluster $P_i$; and
- Figure 8 shows a flowchart algorithm for implementing the flowchart of Figure 7 to clusters $P_i$ of a pre-existing database and for ranking the clusters according to their Z-score value.

Pattern of gene expression in a sample of interest, for example a cell, is characteristic of its current conditions. The conditions may be biological conditions, pathological conditions, or diagnostical or prognostical conditions. The types of cells may also be cultured cells and primary cells isolated from an organism. The effect of a compound or composition on a type of cells may also be assessed using the method of the present invention.

Signals issued from a microarray chip may correspond to measurements of fluorescent intensities. Accordingly, for each gene, there will be as many individual signals as cell types under a given condition.

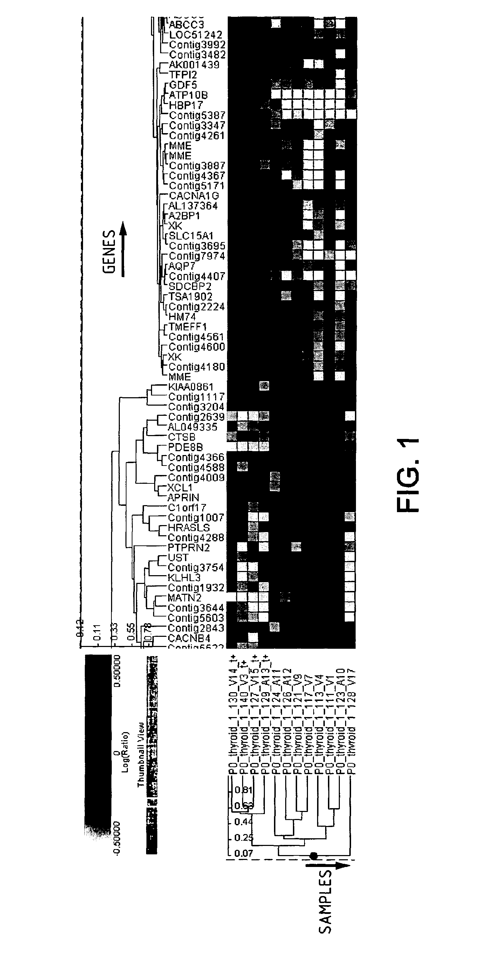

Figure 1 A represents a microarray fluorescence readout consisting of columns of genes (HUGO or Unigene symbols as column name) and rows of samples of interest under different conditions. Complementary DNA (cDNA) were prepared from the samples of interest under condition 0 (the last 8 samples) and condition 1 (the first 4 samples) and were labeled with red and green fluorescence dyes, respectively. Each square corresponds to the fluorescence intensity of a gene in a specific sample. Due to a lack of coloring, the light grey and dark grey squares reflect samples that have been labeled with green and red fluorescent dyes, respectively. This fluorescence is based on the hybridization of the labeled cDNA from said specific sample to the control cDNA that has been affixed onto the microarray chip. Dual-channel laser excites the corresponding dye, which fluoresces in proportion to the degree of hybridization that has occurred. Fluorescence intensity is proportional to the level of gene expression in each sample.

Microarray data may be interpreted within the context of gene function and functional relationship between genes. Usually, microarray data are related to existing biological databases.

The database that can be used in the context of the invention is selected from the group consisting of, for example, the Gene Ontology consortium, the KEGG database or the Biocarta database. Each database gathers genes/gene products that are functionally related into clusters. A gene product might have one or more molecular functions, and may be used in one or more biological process; it might be associated with one or more cellular components. Accordingly, the Gene Ontology consortium that describes how genes products behave in cellular context gathers genes products into clusters according to three principles, i.e. molecular functions, biological processes and cellular components. It is also possible to gather genes from the databases according to their chromosomal localization.

Therefore, it is proposed to seek biological interpretation from microarray experiments directly by searching functions of interest in a whole data set. Instead of being the final step of the analysis, the step of searching functions is considered in the present invention as a starting point to focus on few groups of genes in the rest of the study.

In the context of the present invention, searching biological interpretation from microarray experiments is a freely available tool which allows the identification of pathways and ontologies that contain a relevant number of genes that are differentially expressed between two tested conditions by DNA microarray. The present invention can be applied, for example, to human or mouse studies and makes use of the KEGG pathways, the BioCarta networks and/or the Gene Ontology clusters. The present invention relies on the assessment of p-values associated to t-tests and the calculation of Z-scores after randomization of the genes.

A significance of the results obtained when implementing the invention can be fixed by user. A significance threshold can be chosen preferably between 95% and 99.9 %. Accordingly, the genes that are characterized by a p-value between $5.10^{-2}$ and $10^{-3}$ will be deemed as being significantly differentially expressed. Similarly, a significant Z-score value is superior to 2.5 and more preferably superior to 3, thus indicating that the cluster of selected genes associated to the Z-score contains genes that are significantly differentially expressed.

In figure 1B, pre-existing databases that may be used are the KEGG Pathways, BioCarta Pathways or Gene Ontology. Each database may be organized into groups of gene clusters according to their common involvement in interactive pathways, biological process, molecular function or cellular component. For example, the BioCarta pathways database consists of individual genes, such as G1, G3, G5...Gx or G6, G7...Gy, that are classified into gene clusters according to their common involvement in a pathway, such as pathways x and y, respectively.

The data Set may consist of the log intensities or log ratios that are based on the microarray fluorescence intensity readout shown in Figure 1A. These log intensities or ratios are determined for each gene in order to generate its p-value using a student t-test, or more generally an ANOVA analysis. The p-value corresponds to the probability that a gene is reported as differentially expressed between conditions 0 and 1.

With each pathway of the database, the program of the present invention searches the data set for the genes of the pathway and stores their associated p-value within said pathway. The pathways for which no gene was identified in the data set are suppressed from the rest of the study. Then, for each pathway, the program counts the number of genes found in the data set and the number of genes that have a p-value lower than a predetermined threshold and expressed it as a percentage value (Ps). To test the significance of the percentage of differentially expressed genes by pathways, the program randomly selects X genes from the data set and calculates the percentage of genes with a p-value lower than the threshold compared with X (Pr). This operation is repeated N times (the number N of iterations is preferably determined by the user) and a Z-score is computed for each pathway as followed:

$$Z = \frac{P_s - \overline{P_r}}{\sigma_{P_r}} ,$$

under the null hypothesis H0: Z -N (0,1) wherein $P_s$ is the percentage of significant genes found in the data set for a given annotation; $P_r$ is the percentage of significant genes found randomly, $\overline{P_r}$ the mean of the N$P_r$ and $\sigma_{Pr}$ the square of the variance of the N $P_r$.

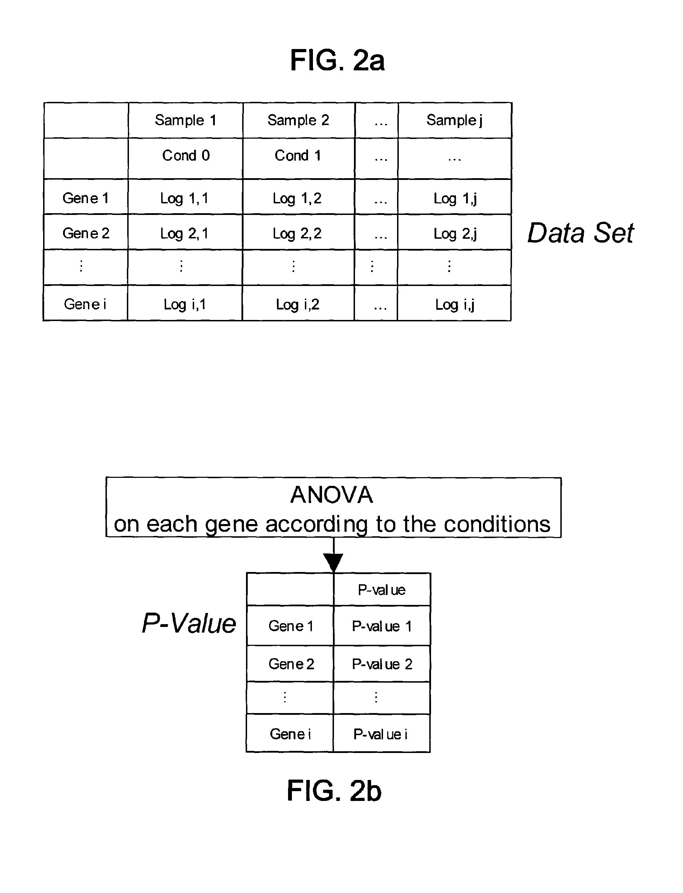

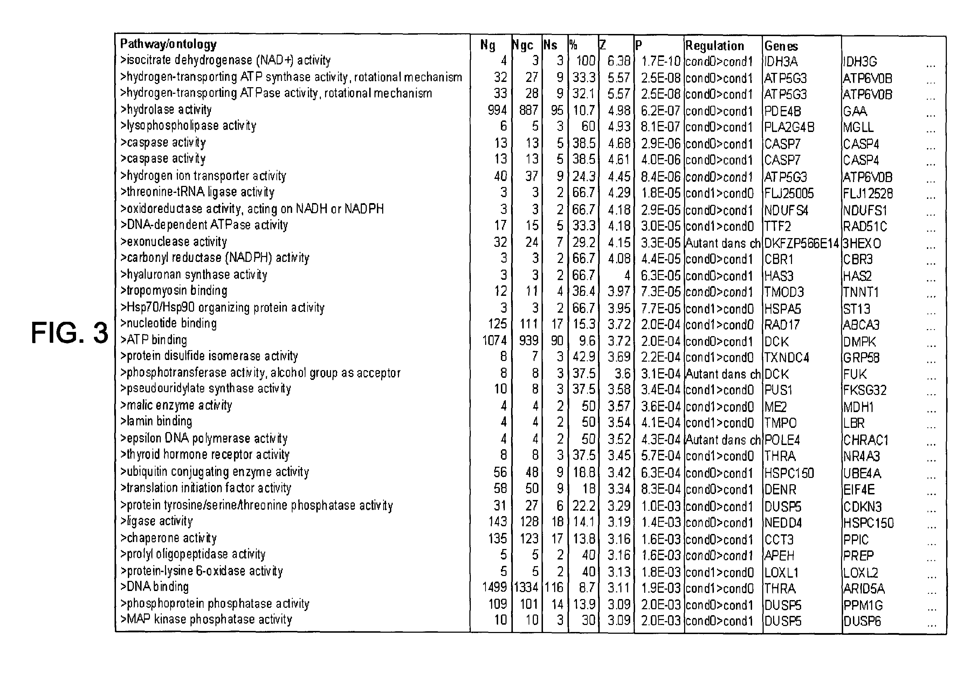

Figures 2a to 2h refer to a first embodiment of the method according to the invention. The method generates a results table such as the one shown in figure 3. On one hand, an expression data set of microarray experiments is collected (figure 2a). Said data set preferably consists of fluorescence intensities that are obtained from the microarray fluorescence read out (figure 1A) and may be expressed either in log(ratio), ratio, log(intensity), fold-change or any numerical expression related to the fluorescence. Missing values or unusable experimental values, in the submitted data set, can be represented by a blank value. It is possible to choose how many *"blank values"* per row can be authorized and keep the corresponding gene within the analysis. For a given gene, if the number of "*blank values*" is over a threshold, this given gene will not be taken into account; if it is lower, a t-test or ANOVA will be performed on the non missing values. For each gene that is kept in the analysis, the method according to the present invention performs a statistical test (either ANOVA or t-test in the case of two conditions) that compares the mean of expression level between the studied conditions. ANOVA analysis tests whether the null hypothesis that the difference between the means of expression level in each condition is zero. If the difference between the means is among those that would occur rarely by chance when the null hypothesis is true, the null hypothesis is rejected and the results is considered as statistically significant. Using said test, each gene is assigned to a p-value in order to ascertain whether said gene is significantly differentially expressed between the tested conditions (figure 2b).

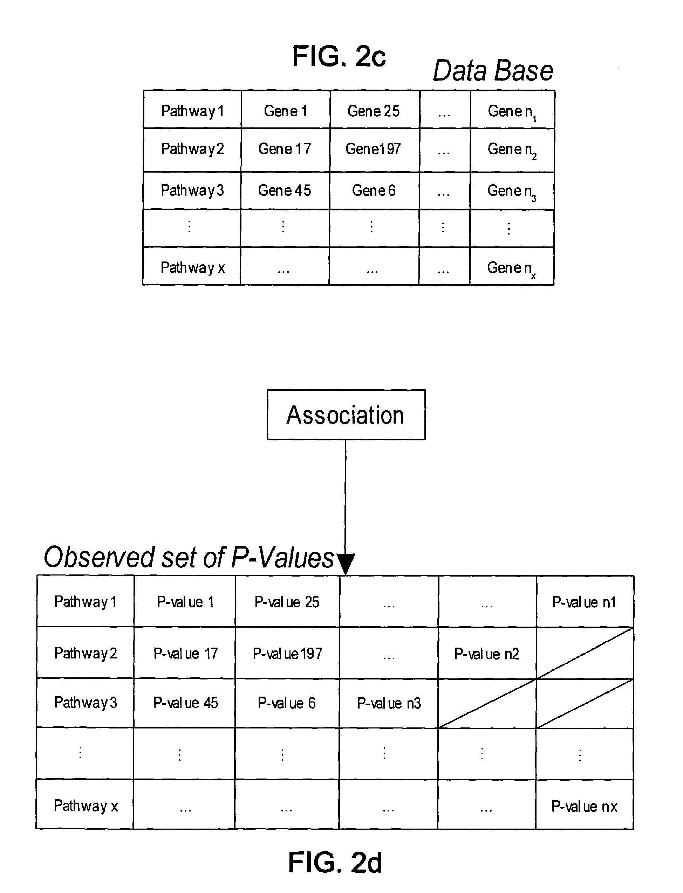

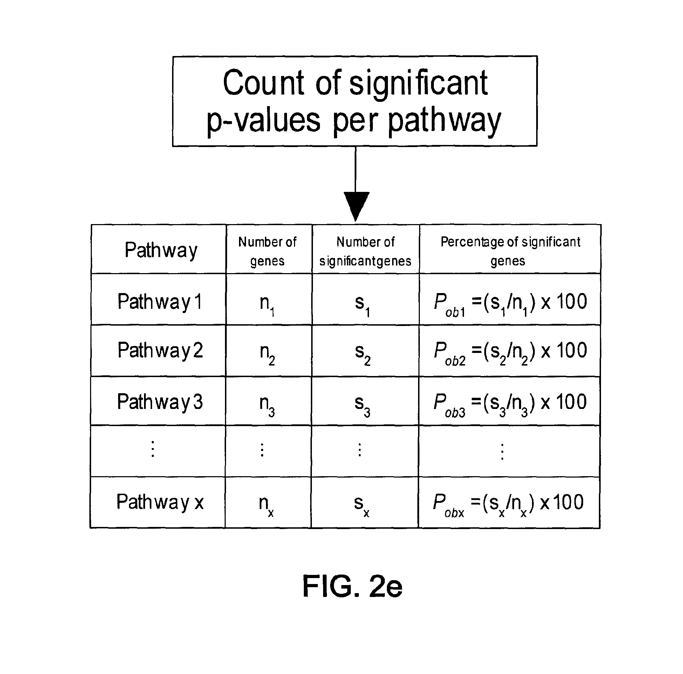

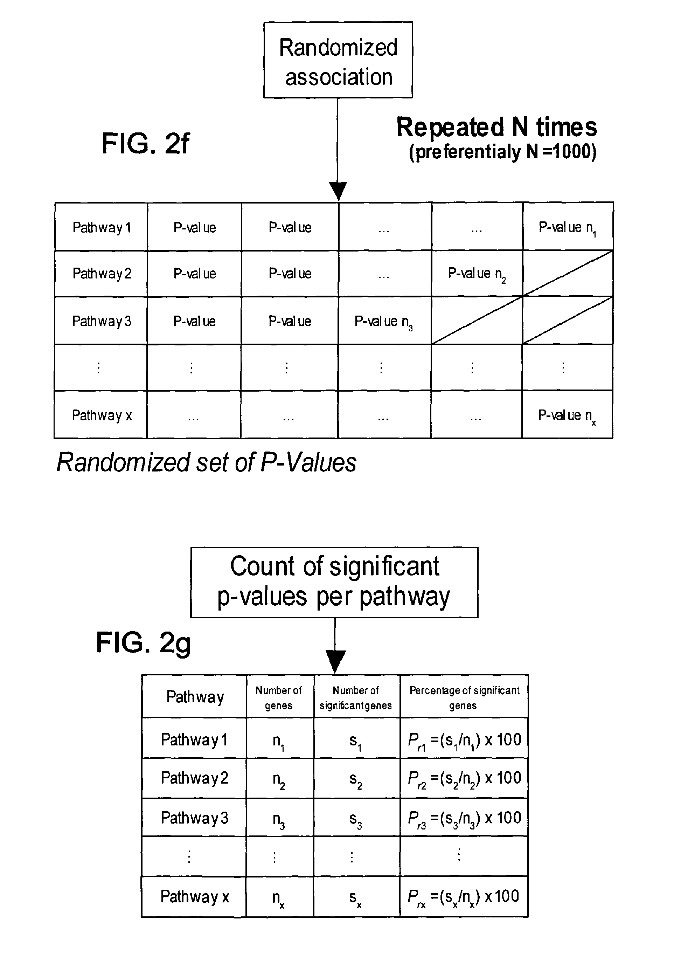

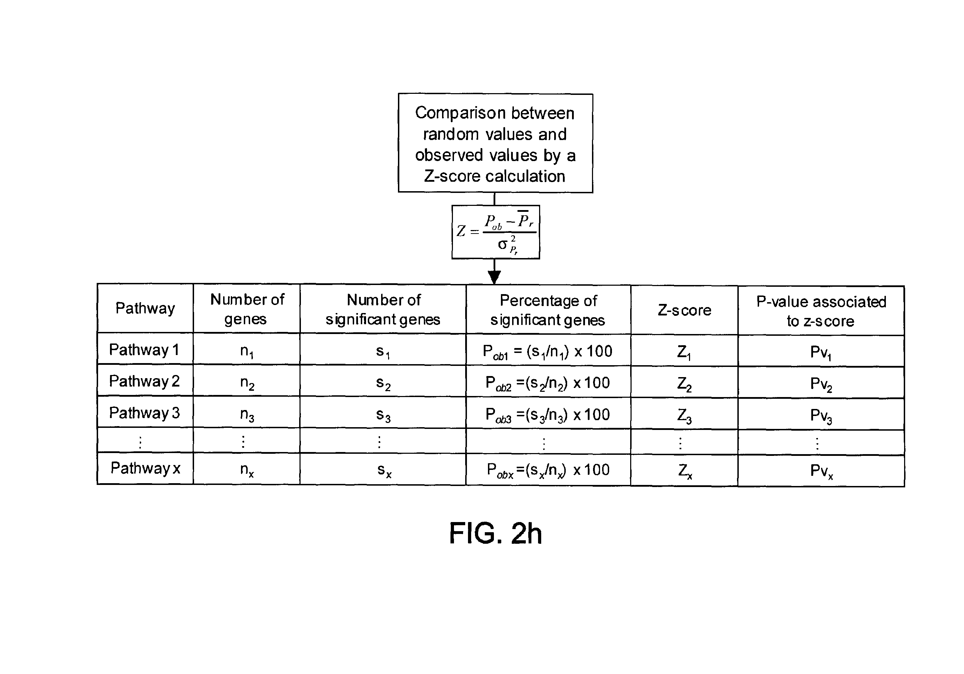

On the other hand, files containing information from database such as KEGG, Biocarta and Gene Ontology are collected (figure 2c). These files are automatically updated. For each cluster of genes (of an ontology term or pathway), the program according to the present invention searches the genes that are present in both said cluster and the submitted data set (figure 2d). Then, the program counts for each cluster the number of these genes that have an associated p-value lower than a predetermined threshold (fixed-by-user), thus identifying the genes of the cluster that are differentially expressed between the tested conditions. When the program is applied to each cluster of a given database, the clusters that have a significant number of differentially expressed genes are identified (figure 2e). Then, for X genes in a given cluster X, a step of the method according to the invention consists of picking out randomly X genes in the data set (as shown in the example of figure 2b) and, counting how many of these genes are differentially expressed (figure 2f). This step is repeated N times, preferably a thousand times (N=1000). Then, clusters that have a significant number of genes differentially expressed at random are known (figure 2g). Finally, for each given cluster, a Z-score (figure 2h) is calculated by comparing the observed values (figure 2e) to the randomized values (figure 2g).

Figure 3 shows a representative results table consisting of a rank list of clusters from pre-existing database. In the example of the present results table, genes were clustered according to their cellular functions or activities (pathway names listed in the box) within the Gene Ontology database with only two genes of each listed (right box). Each pathway cluster has a calculated Z-score (Figure 1 B) that is used to rank them in decreasing order of value. Only the pathway clusters with Z-scores that are greater than a predetermined threshold, having preferably a value of 3 in the given example, are deemed to be significant. The pathway cluster with the highest Z-score is the most significant, which indicates that it has the most differential expression between conditions 0 and 1.

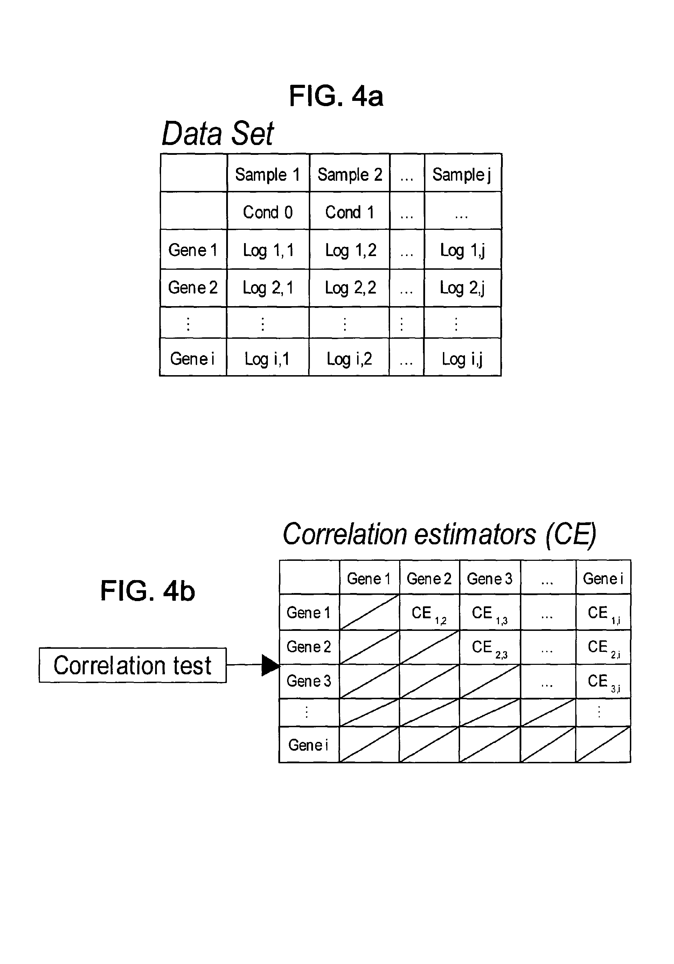

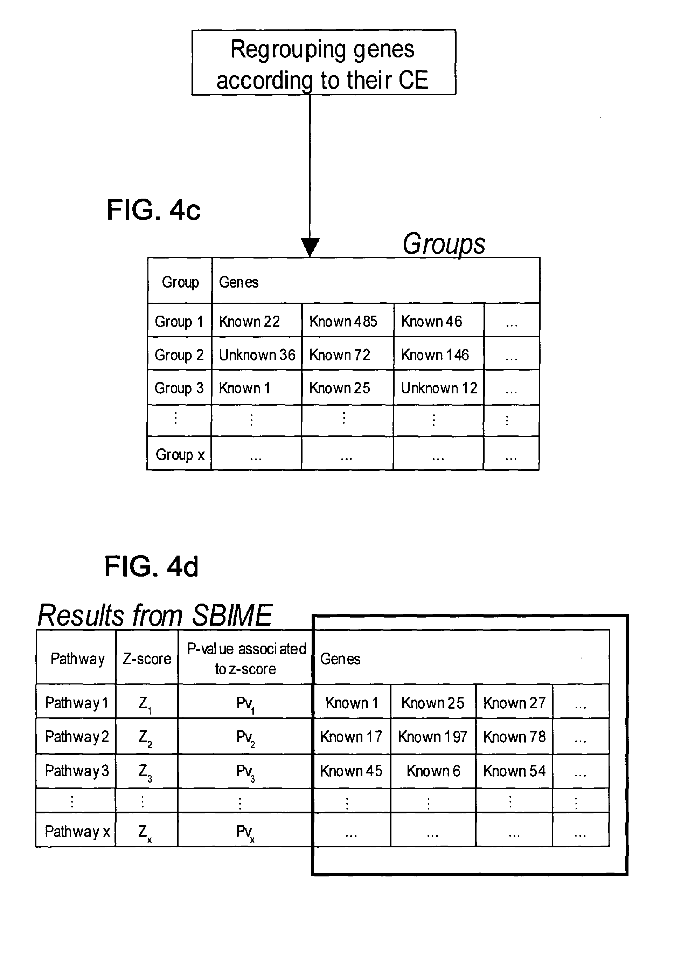

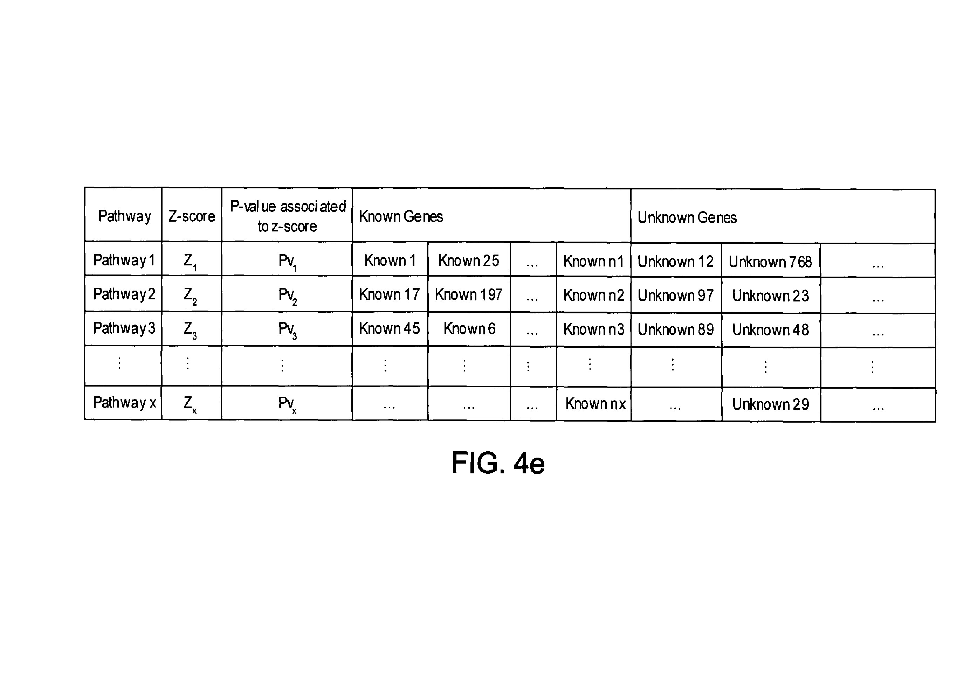

Figures 4a to 4e refer to a second embodiment of the method according to the present invention, which allows the determination of a function of an unknown gene as described hereafter. From the data set (figure 4a), which is preferably the same than the data set of figure 2a, the software calculates correlation estimators between each possible pair of genes (figure 4b) using a correlation test. The correlation estimator may either be a distance or a correlation coefficient, or any estimator of the similarity in expression level between two genes. Thanks to these correlation estimators, groups of genes are clustered according to the similarity in their expression level (figure 4c). The method according to this embodiment may also combine different types of correlation estimators (figure 4b), such as distances and correlation coefficients to group genes according to different criteria (figure 4c). In any cases, associations are made between the results (figure 4d) and the groups obtained (as shown in figure 4c) in order to assign unknown genes (figure 4e) to clusters of known genes (the pathways shown in the example of figure 4e) that share similar level of expression (in figure 4c). Accordingly, the method according to this embodiment assigns putative functions, processes, interactions to unknown genes or pathways (as shown in figure 4e).

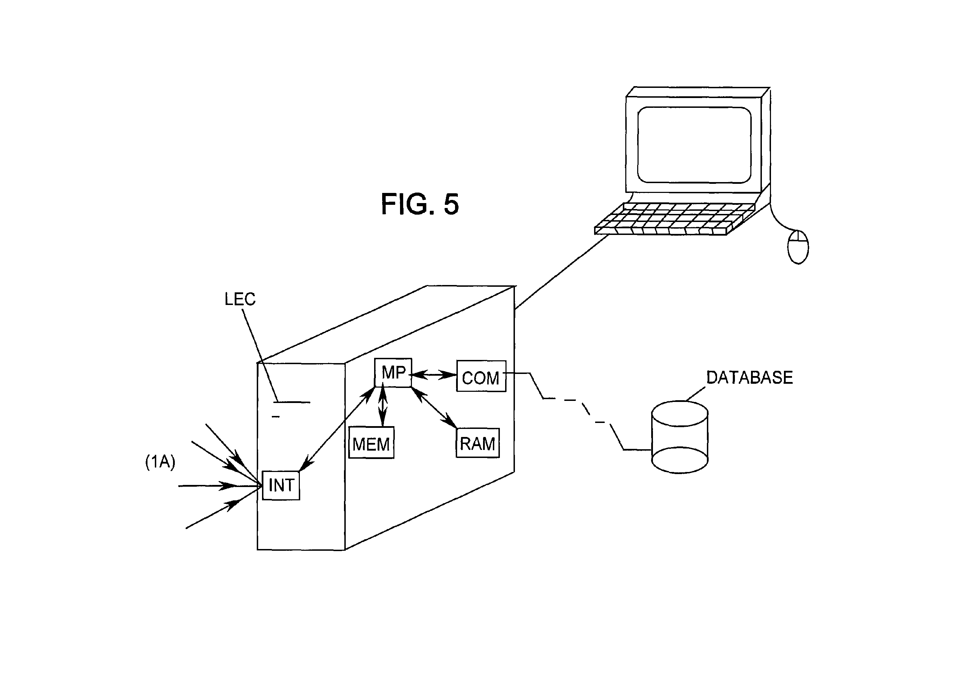

A system for performing the invention is shown in Figure 5. Said system includes a processing unit PU composed of:

- an interface INT for receiving signals issued from the microarray data set,
- a processor MP,
- a memory for storing results and the signals measurements obtained from the microarray,
- a working memory RAM (random access memory).

More specifically, the memory MEM is arranged for storing a computer program product in order to perform the steps according to the method of the invention. The software may also be loaded from a CD-ROM player and/or from the internet. In addition, the system may also include a communication interface COM, such as a modem or an equivalent device in order to import information from pre-existing databases.

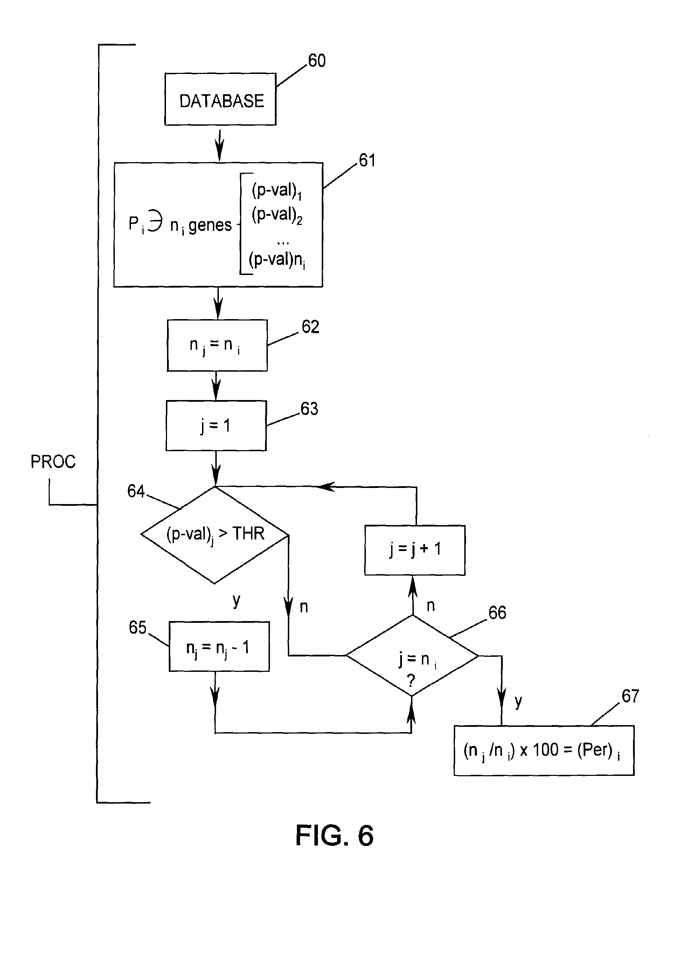

Figure 6 shows a representative flowchart of an embodiment of the algorithm of the software according to the invention. In step 60, the software activates a connection to a pre-existing database DB to download a number of $n_i$ genes that are involved in a cluster $P_i$. The cluster $P_i$ may be a pathway, a function, a process or a cellular component. In step 61, a p-value is determined for each of the $n_i$ genes involved in the cluster $P_i$ as described in Figure 1 B. In step 62, the software assigns a variable $n_j$ that is equal to the number of $n_i$ genes involved in the cluster $P_i$. In step 63, the value of a floating variable j is set at j=1 and a recurrent treatment is implemented for the floating variable j. The test 64 compares the p-value $(p\text{-}val)_j$ of gene $G_j$ of the cluster $P_i$ with a predetermined threshold THR. For example, the value of the threshold may be equal to $10^{-3}$. If the p-value $(p\text{-}val)_j$ is greater than said threshold THR (arrow y), the floating variable $n_j$ as defined in step 62 is decremented by one unit. If the p-value $(p\text{-}val)_j$ is significant, i.e. less than the threshold THR, step 66 verifies whether the floating variable j reaches the value of the number of genes $n_i$ of the cluster $P_i$. If test 66 is positive (arrow y), the percentage

$(Per)_i$ of genes that have a significant p-value in the cluster $P_i$ is calculated in step 67. Otherwise (if variable j has not reached the number $n_i$ : arrow n), the value of the floating variable j is incremented and step 64 is repeated.

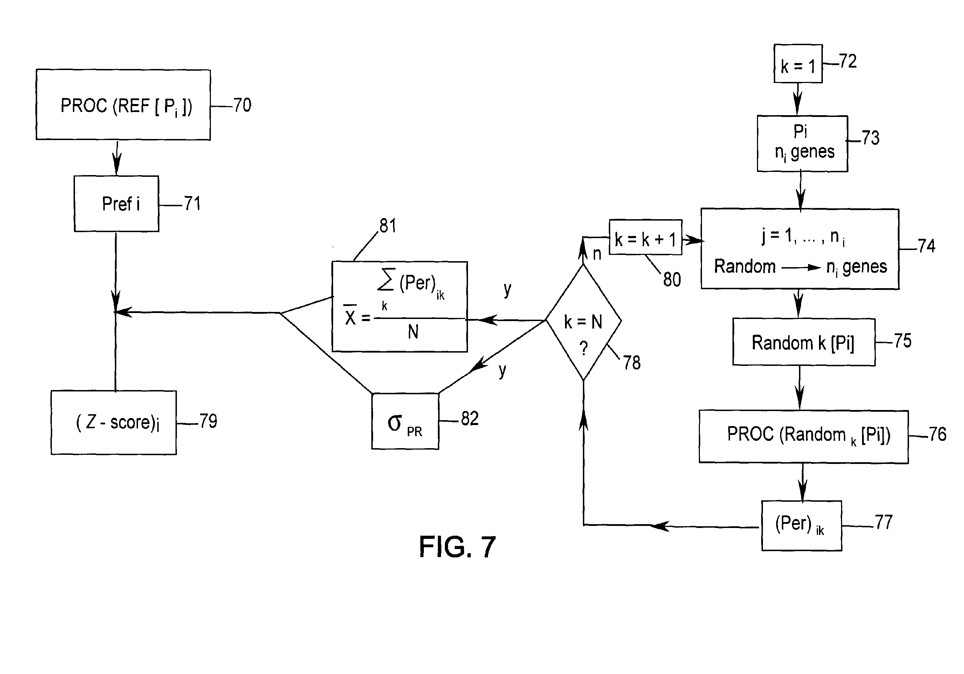

The flowchart algorithm shown in Figure 6 consists of a main routine PROC that is intended to be applied to both the genes involved in a cluster from a preexisting database and the randomly drawn genes. Therefore the Z-score value $(z\text{-}score)_i$ assigned to each cluster $P_i$ can be calculated by using a software having a flowchart algorithm as represented in Figure 7. Step 70 of Figure 7 consists of applying the main routine PROC to the cluster $P_i$ from a pre-existing database with the exact genes given by the database $REF[P_i]$. In step 71, a percentage $Pref_i$ is obtained by running the main routine PROC as previously described with reference to Figure 6. On the other hand, a floating variable k is set in step 72. The floating variable k corresponds to an index of random draws amongst N=1000 draws in the given example. In step 73, the number of $n_i$ genes that are involved in the cluster $P_i$ is obtained from the database. In step 74, $n_i$ genes are randomly drawn. In step 75, the p-value of each randomly drawn gene is used to perform the main routine PROC on the $n_i$ randomly drawn genes (step 76). Then, the percentage of genes amongst the randomly drawn genes that have a significant p-value is determined as previously described with reference to Figure 6. Test 78 verifies whether the number of draws reaches a value of N=1000. If the floating variable k equals to N (arrow y), a Z-score value is calculated and assigned to each cluster $P_i$ in step 79. Otherwise (arrow n), the value of the floating variable k is incremented. Each Z-score value is calculated according to the formula:

$$(z - score)_i = \frac{Pref_i - \overline{X}}{\sigma_{PR}},$$

wherein:

$$\overline{X} = \frac{1}{N}\sum_k (Per)_{ik},$$

representing the mean of the N $(Per)_{ik}$ values over the N draws (step 81), and $\sigma_{PR}$ is related to the variance of the draws (step 82).

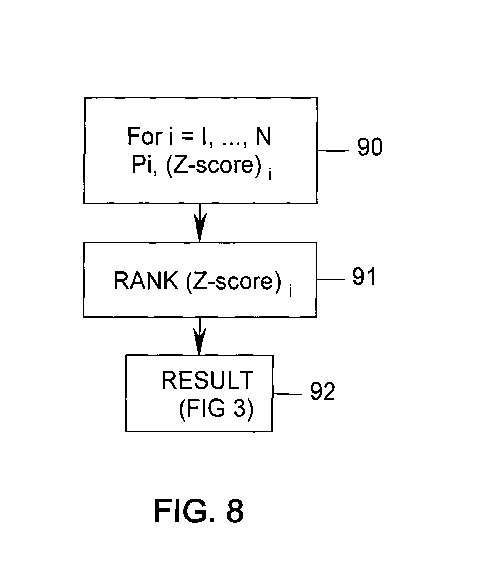

Referring to Figure 8, the flowchart algorithm that is represented in Figure 7 is applied to all the clusters $P_i$ obtained from the pre-existing database. In step 90, a Z-score value is calculated and assigned to each cluster $P_i$. In step 91, the clusters $P_i$ are ranked according to their respective Z-score value. The clusters that have a Z-score value greater than a predetermined threshold are selected as significant. In step 92, a results table such as the one represented in Figure 3 is finally obtained.

The results obtained by implementing the present invention have been validated by laboratory experiments, in particular on human and mouse samples. The tests show that the bigger the submitted microarray data sets are, the more relevant the results obtained by the present invention are.

**Claims**

1. Method implemented by computer means for analyzing DNA microarray data given by a DNA microarray set that generates:

   - for a plurality of genes, on one hand,
   - and for a plurality of cell types under different biological and/or pathological condition, on the other hand,

   signals corresponding to an expression level of a gene in a cell type under a predetermined condition,

   said process comprising:

   a) for each gene, on the basis of said signals, determining a statistical parameter that corresponds to a probability that said gene is reported as being differentially expressed between said plurality of cell types and under said predetermined condition,
   b) assigning to each gene of the microarray at least one cluster of a pre-existing database, said cluster containing a given number of genes of the microarray, and
   c) for each cluster, selecting the genes based on a significance of their statistical parameter and determining a number of the selected genes,

   wherein the method further comprises the steps of:

   d) randomly draw N times a given number of genes as defined in step b) from the microarray and associate to each of said random genes their statistical parameter for determining, for each draw, a number of random genes that have a significant statistical parameter, and evaluate a mean value, over the N draws, of said numbers of random genes that have a significant statistical parameter,
   e) compare said mean value with said number of selected genes as determined in step c), a result of said comparison indicating a significance of said cluster.

2. The method according to claim 1, wherein the number N is approximately one thousand.

3. The method according to claim 1 or claim 2, wherein said comparison of step e) comprises a Z-score calculation according to a formula:

$$Z_i = \frac{P_s - \overline{P_r}}{\sigma_{P_r}} \quad,$$

wherein:

   - $P_s$ is a percentage of significant genes selected in the microarray for a given cluster i;
   - $\overline{P_r}$ is a mean value, over N draws, of N percentages $P_r$ of randomly drawn significant genes, and
   - $\sigma_{P_r}$ is the square of a variance of said N percentages $P_r$.

4. The method according to any of the precedent claims, wherein the selection of step c) is based on a comparison of the statistical parameter of each gene and a threshold value.

5. The method according to any of the precedent claims, wherein said statistical parameter is a p-value in an analysis of variance such as ANOVA.

6. The method according to any of the precedent claims, wherein said statistical parameter is a p-value in a mean comparison analysis such as a Student t-test.

7. The method according to claim 4, taken in combination with any of claims 5 and 6, wherein a selection of a gene in step c) is based on whether its p-value is inferior to a threshold value.

8. The method according to claim 7, wherein said threshold value is preferably $10^{-3}$.

9. The method according to any of the precedent claims, wherein:

   - said pre-existing database comprises a plurality of clusters, each cluster comprising a given number of genes of the microarray,
   - steps d) and e) are performed for each cluster, the method further comprising a step of:

   f) ranking the clusters of said plurality of clusters with respect to their comparison results.

10. The method according to claim 9, wherein a selection of the clusters that have a comparison result which is higher than a predetermined threshold, as the clusters the most significant, is performed at step f).

11. The method according to claim 10, wherein said predetermined threshold is fixed by user.

12. The method according to any of claims 10 and 11, taken in combination with claim 3, wherein the ranking of step f) is performed on Z-score values associated to clusters of said plurality of clusters, respectively, and wherein said most significant clusters have a Z-score value, as a comparison result, which is higher than said predetermined threshold.

13. The method of claim 12, wherein said Z-score threshold value is preferably fixed at 3.

14. The method according to any of the precedent claims, wherein:

- in step a), a correlation test is further performed on each pair of genes of the microarray and a correlation estimator is calculated for each pair of genes,
- groups of genes having a high correlation estimator between them are clustered, and
- the content of a cluster given by the pre-existing database in step b) is enhanced with at least one unknown gene which belongs to a same group than a known gene already indexed in said given cluster of said database.

15. The method according to claim 14, wherein said correlation estimator represents a distance between the expression levels of two genes of a pair.

16. The method according to claim 15, wherein said groups are clustered with respect to a similarity criteria between expression levels of two genes.

17. The method according to any of the precedent claims, wherein said signals are derived from florescence intensity measurements.

18. The method according to any of the precedent claims, wherein said clusters are related to metabolic pathways and/or molecular functions and/or interaction networks and/or biological processes and/or cellular components, and/or chromosomal localization.

19. A computer system for performing the method according to any of the precedent claims, comprising:

- an interface module for collecting the signals issued from a DNA microarray set,
- a communication module for obtaining said clusters from said pre-existing database,
- a unit for processing said signals, and
- a memory medium for storing a computer program intended to be run by said processing unit for performing all or some of the steps of the method according to any of the precedent claims.

20. A computer program product intended to be stored in a memory of a processor unit or on a removable memory medium suitable for co-operating with a reader of said processor unit, or downloadable from a distant site, wherein the program product comprises instructions for implementing all or some of the steps of the method according to any of claims 1 to 18.

FIG. 1

## FIG. 2a

| | Sample 1 | Sample 2 | ... | Sample j | |
|---|---|---|---|---|---|
| | Cond 0 | Cond 1 | ... | ... | |
| Gene 1 | Log 1,1 | Log 1,2 | ... | Log 1,j | *Data Set* |
| Gene 2 | Log 2,1 | Log 2,2 | ... | Log 2,j | |
| ⋮ | ⋮ | ⋮ | ⋮ | ⋮ | |
| Gene i | Log i,1 | Log i,2 | ... | Log i,j | |

| ANOVA |
|---|
| on each gene according to the conditions |

*P-Value*

| | P-value |
|---|---|
| Gene 1 | P-value 1 |
| Gene 2 | P-value 2 |
| ⋮ | ⋮ |
| Gene i | P-value i |

## FIG. 2b

# FIG. 2c

*Data Base*

| | | | | |
|---|---|---|---|---|
| Pathway 1 | Gene 1 | Gene 25 | ... | Gene $n_1$ |
| Pathway 2 | Gene 17 | Gene 197 | ... | Gene $n_2$ |
| Pathway 3 | Gene 45 | Gene 6 | ... | Gene $n_3$ |
| ⋮ | ⋮ | ⋮ | ⋮ | ⋮ |
| Pathway x | ... | ... | ... | Gene $n_x$ |

Association

*Observed set of P-Values* ▼

| | | | | | |
|---|---|---|---|---|---|
| Pathway 1 | P-value 1 | P-value 25 | ... | ... | P-value n1 |
| Pathway 2 | P-value 17 | P-value 197 | ... | P-value n2 | |
| Pathway 3 | P-value 45 | P-value 6 | P-value n3 | | |
| ⋮ | ⋮ | ⋮ | ⋮ | ⋮ | ⋮ |
| Pathway x | ... | ... | ... | ... | P-value nx |

# FIG. 2d

| Count of significant p-values per pathway |
|---|

| Pathway | Number of genes | Number of significant genes | Percentage of significant genes |
|---|---|---|---|
| Pathway 1 | $n_1$ | $s_1$ | $P_{ob1} = (s_1/n_1) \times 100$ |
| Pathway 2 | $n_2$ | $s_2$ | $P_{ob2} = (s_2/n_2) \times 100$ |
| Pathway 3 | $n_3$ | $s_3$ | $P_{ob3} = (s_3/n_3) \times 100$ |
| $\vdots$ | $\vdots$ | $\vdots$ | $\vdots$ |
| Pathway x | $n_x$ | $s_x$ | $P_{obx} = (s_x/n_x) \times 100$ |

## FIG. 2e

**Randomized association**

**FIG. 2f**

**Repeated N times**
**(preferentialy N =1000)**

| Pathway 1 | P-value | P-value | ... | ... | P-value $n_1$ |
|---|---|---|---|---|---|
| Pathway 2 | P-value | P-value | ... | P-value $n_2$ | |
| Pathway 3 | P-value | P-value | P-value $n_3$ | | |
| ⋮ | ⋮ | ⋮ | ⋮ | ⋮ | ⋮ |
| Pathway x | ... | ... | ... | ... | P-value $n_x$ |

*Randomized set of P-Values*

**Count of significant p-values per pathway**

**FIG. 2g**

| Pathway | Number of genes | Number of significant genes | Percentage of significant genes |
|---|---|---|---|
| Pathway 1 | $n_1$ | $s_1$ | $P_{r1} = (s_1/n_1) \times 100$ |
| Pathway 2 | $n_2$ | $s_2$ | $P_{r2} = (s_2/n_2) \times 100$ |
| Pathway 3 | $n_3$ | $s_3$ | $P_{r3} = (s_3/n_3) \times 100$ |
| ⋮ | ⋮ | ⋮ | ⋮ |
| Pathway x | $n_x$ | $s_x$ | $P_{rx} = (s_x/n_x) \times 100$ |

Comparison between
random values and
observed values by a
Z-score calculation

$$Z = \frac{P_{ob} - \overline{P_r}}{\sigma_{P_r}^2}$$

| Pathway | Number of genes | Number of significant genes | Percentage of significant genes | Z-score | P-value associated to z-score |
|---|---|---|---|---|---|
| Pathway 1 | $n_1$ | $s_1$ | $P_{ob1} = (s_1/n_1) \times 100$ | $Z_1$ | $Pv_1$ |
| Pathway 2 | $n_2$ | $s_2$ | $P_{ob2} = (s_2/n_2) \times 100$ | $Z_2$ | $Pv_2$ |
| Pathway 3 | $n_3$ | $s_3$ | $P_{ob3} = (s_3/n_3) \times 100$ | $Z_3$ | $Pv_3$ |
| ⋮ | ⋮ | ⋮ | ⋮ | ⋮ | ⋮ |
| Pathway x | $n_x$ | $s_x$ | $P_{obx} = (s_x/n_x) \times 100$ | $Z_x$ | $Pv_x$ |

FIG. 2h

FIG. 3

| Pathway/ontology | Ng | Ngc | Ns | % | Z | P | Regulation | Genes | | |
|---|---|---|---|---|---|---|---|---|---|---|
| >isocitrate dehydrogenase (NAD+) activity | 4 | 3 | 3 | 100 | 6.38 | 1.7E-10 | cond0>cond1 | IDH3A | IDH3G | ... |
| >hydrogen-transporting ATP synthase activity, rotational mechanism | 32 | 27 | 9 | 33.3 | 5.57 | 2.5E-08 | cond0>cond1 | ATP5G3 | ATP6V0B | ... |
| >hydrogen-transporting ATPase activity, rotational mechanism | 33 | 28 | 9 | 32.1 | 5.57 | 2.5E-08 | cond0>cond1 | ATP5G3 | ATP6V0B | ... |
| >hydrolase activity | 994 | 887 | 95 | 10.7 | 4.98 | 6.2E-07 | cond0>cond1 | PDE4B | GAA | ... |
| >lysophospholipase activity | 6 | 5 | 3 | 60 | 4.93 | 8.1E-07 | cond0>cond1 | PLA2G4B | MGLL | ... |
| >caspase activity | 13 | 13 | 5 | 38.5 | 4.68 | 2.9E-06 | cond0>cond1 | CASP7 | CASP4 | ... |
| >caspase activity | 13 | 13 | 5 | 38.5 | 4.61 | 4.0E-06 | cond0>cond1 | CASP7 | CASP4 | ... |
| >hydrogen ion transporter activity | 40 | 37 | 9 | 24.3 | 4.45 | 8.4E-06 | cond0>cond1 | ATP5G3 | ATP6V0B | ... |
| >threonine-tRNA ligase activity | 3 | 3 | 2 | 66.7 | 4.29 | 1.8E-05 | cond1>cond0 | FLJ25005 | FLJ12528 | ... |
| >oxidoreductase activity, acting on NADH or NADPH | 3 | 3 | 2 | 66.7 | 4.18 | 2.9E-05 | cond0>cond1 | NDUFS4 | NDUFS1 | ... |
| >DNA-dependent ATPase activity | 17 | 15 | 5 | 33.3 | 4.18 | 3.0E-05 | cond1>cond0 | TTF2 | RAD51C | ... |
| >exonuclease activity | 32 | 24 | 7 | 29.2 | 4.15 | 3.3E-05 | Autant dans ch | DKFZP566E14 | 3HEXO | ... |
| >carbonyl reductase (NADPH) activity | 3 | 3 | 2 | 66.7 | 4.08 | 4.4E-05 | cond0>cond1 | CBR1 | CBR3 | ... |
| >hyaluronan synthase activity | 3 | 3 | 2 | 66.7 | 4 | 6.3E-05 | cond0>cond1 | HAS3 | HAS2 | ... |
| >tropomyosin binding | 12 | 11 | 4 | 36.4 | 3.97 | 7.3E-05 | cond0>cond1 | TMOD3 | TNNT1 | ... |
| >Hsp70/Hsp90 organizing protein activity | 3 | 3 | 2 | 66.7 | 3.95 | 7.7E-05 | cond1>cond0 | HSPA5 | ST13 | ... |
| >nucleotide binding | 125 | 111 | 17 | 15.3 | 3.72 | 2.0E-04 | cond1>cond0 | RAD17 | ABCA3 | ... |
| >ATP binding | 1074 | 939 | 90 | 9.6 | 3.72 | 2.0E-04 | cond0>cond1 | DCK | DMPK | ... |
| >protein disulfide isomerase activity | 8 | 7 | 3 | 42.9 | 3.69 | 2.2E-04 | cond1>cond0 | TXNDC4 | GRP58 | ... |
| >phosphotransferase activity, alcohol group as acceptor | 8 | 8 | 3 | 37.5 | 3.6 | 3.1E-04 | Autant dans ch | DCK | FUK | ... |
| >pseudouridylate synthase activity | 10 | 8 | 3 | 37.5 | 3.58 | 3.4E-04 | cond1>cond0 | PUS1 | FKSG32 | ... |
| >malic enzyme activity | 4 | 4 | 2 | 50 | 3.57 | 3.6E-04 | cond1>cond0 | ME2 | MDH1 | ... |
| >lamin binding | 4 | 4 | 2 | 50 | 3.54 | 4.1E-04 | cond1>cond0 | TMPO | LBR | ... |
| >epsilon DNA polymerase activity | 4 | 4 | 2 | 50 | 3.52 | 4.3E-04 | Autant dans ch | POLE4 | CHRAC1 | ... |
| >thyroid hormone receptor activity | 8 | 8 | 3 | 37.5 | 3.45 | 5.7E-04 | cond1>cond0 | THRA | NR4A3 | ... |
| >ubiquitin conjugating enzyme activity | 56 | 48 | 9 | 16.8 | 3.42 | 6.3E-04 | cond0>cond1 | HSPC150 | UBE4A | ... |
| >translation initiation factor activity | 58 | 50 | 9 | 18 | 3.34 | 8.3E-04 | cond0>cond1 | DENR | EIF4E | ... |
| >protein tyrosine/serine/threonine phosphatase activity | 31 | 27 | 6 | 22.2 | 3.29 | 1.0E-03 | cond0>cond1 | DUSP5 | CDKN3 | ... |
| >ligase activity | 143 | 128 | 18 | 14.1 | 3.19 | 1.4E-03 | cond0>cond1 | NEDD4 | HSPC150 | ... |
| >chaperone activity | 135 | 123 | 17 | 13.8 | 3.16 | 1.6E-03 | cond0>cond1 | CCT3 | PPIC | ... |
| >prolyl oligopeptidase activity | 5 | 5 | 2 | 40 | 3.16 | 1.6E-03 | cond0>cond1 | APEH | PREP | ... |
| >protein-lysine 6-oxidase activity | 5 | 5 | 2 | 40 | 3.13 | 1.8E-03 | cond1>cond0 | LOXL1 | LOXL2 | ... |
| >DNA binding | 1499 | 1334 | 116 | 8.7 | 3.11 | 1.9E-03 | cond1>cond0 | THRA | ARID5A | ... |
| >phosphoprotein phosphatase activity | 109 | 101 | 14 | 13.9 | 3.09 | 2.0E-03 | cond0>cond1 | DUSP5 | PPM1G | ... |
| >MAP kinase phosphatase activity | 10 | 10 | 3 | 30 | 3.09 | 2.0E-03 | cond0>cond1 | DUSP5 | DUSP6 | ... |

# FIG. 4a

## Data Set

|  | Sample 1 | Sample 2 | ... | Sample j |
|---|---|---|---|---|
|  | Cond 0 | Cond 1 | ... | ... |
| Gene 1 | Log 1,1 | Log 1,2 | ... | Log 1,j |
| Gene 2 | Log 2,1 | Log 2,2 | ... | Log 2,j |
| ⋮ | ⋮ | ⋮ | ⋮ | ⋮ |
| Gene i | Log i,1 | Log i,2 | ... | Log i,j |

## Correlation estimators (CE)

# FIG. 4b

Correlation test →

|  | Gene 1 | Gene 2 | Gene 3 | ... | Gene i |
|---|---|---|---|---|---|
| Gene 1 |  | $CE_{1,2}$ | $CE_{1,3}$ | ... | $CE_{1,i}$ |
| Gene 2 |  |  | $CE_{2,3}$ | ... | $CE_{2,i}$ |
| Gene 3 |  |  |  | ... | $CE_{3,i}$ |
| ⋮ |  |  |  |  | ⋮ |
| Gene i |  |  |  |  |  |

Regrouping genes
according to their CE

## FIG. 4c

*Groups*

| Group | Genes | | | |
|---|---|---|---|---|
| Group 1 | Known 22 | Known 485 | Known 46 | ... |
| Group 2 | Unknown 36 | Known 72 | Known 146 | ... |
| Group 3 | Known 1 | Known 25 | Unknown 12 | ... |
| ⋮ | ⋮ | ⋮ | ⋮ | ⋮ |
| Group x | ... | ... | ... | ... |

## FIG. 4d

*Results from SBIME*

| Pathway | Z-score | P-value associated to z-score | Genes | | | |
|---|---|---|---|---|---|---|
| Pathway 1 | $Z_1$ | $Pv_1$ | Known 1 | Known 25 | Known 27 | ... |
| Pathway 2 | $Z_2$ | $Pv_2$ | Known 17 | Known 197 | Known 78 | ... |
| Pathway 3 | $Z_3$ | $Pv_3$ | Known 45 | Known 6 | Known 54 | ... |
| ⋮ | ⋮ | ⋮ | ⋮ | ⋮ | ⋮ | ⋮ |
| Pathway x | $Z_x$ | $Pv_x$ | ... | ... | ... | ... |

| Pathway | Z-score | P-value associated to z-score | Known Genes | | | | Unknown Genes | | |
|---|---|---|---|---|---|---|---|---|---|
| Pathway 1 | $Z_1$ | $Pv_1$ | Known 1 | Known 25 | ... | Known n1 | Unknown 12 | Unknown 768 | ... |
| Pathway 2 | $Z_2$ | $Pv_2$ | Known 17 | Known 197 | ... | Known n2 | Unknown 97 | Unknown 23 | ... |
| Pathway 3 | $Z_3$ | $Pv_3$ | Known 45 | Known 6 | ... | Known n3 | Unknown 89 | Unknown 48 | ... |
| ⋮ | ⋮ | ⋮ | ⋮ | ⋮ | ⋮ | ⋮ | ⋮ | ⋮ | ⋮ |
| Pathway x | $Z_x$ | $Pv_x$ | ... | ... | ... | Known nx | ... | Unknown 29 | ... |

## FIG. 4e

EP 1 691 311 A1

FIG. 5

LEC

(1A)

INT

MEM

MP

RAM

COM

DATABASE

EP 1 691 311 A1

DATABASE ⟶ 60

$P_i \ni n_i$ genes $\left[\begin{array}{l} (p\text{-val})_1 \\ (p\text{-val})_2 \\ ... \\ (p\text{-val})n_i \end{array}\right.$ ⟶ 61

$n_j = n_i$ ⟶ 62

$j = 1$ ⟶ 63

64 — $(p\text{-val})_j > THR$

PROC

y

65 — $n_j = n_j - 1$

n

$j = j + 1$

n

$j = n_i$ ? ⟶ 66

y

$(n_j / n_i) \times 100 = (Per)_i$ ⟶ 67

**FIG. 6**

FIG. 7

For i = I, ..., N
$P_i, (Z\text{-score})_i$ —— 90

RANK $(Z\text{-score})_i$ —— 91

RESULT
(FIG 3) —— 92

FIG. 8

**European Patent Office**

**EUROPEAN SEARCH REPORT**

Application Number

EP 05 29 0333

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | TUSHER V G ET AL: "Significance analysis of microarrays applied to the ionizing radition response" PROCEEDINGS OF THE NATIONAL ACADEMY OF SCIENCES OF USA, NATIONAL ACADEMY OF SCIENCE. WASHINGTON, US, vol. 98, no. 9, 24 April 2001 (2001-04-24), pages 5116-5121, XP002967440 ISSN: 0027-8424 | 19,20 | G06F19/00 |
| A | * abstract * * page 5116, left-hand column * * page 5119, right-hand column, last paragraph - page 5121, right-hand column, last paragraph * | 1-18 | |
| A | JENSSEN T-K ET AL: "A literature network of human genes for high-throughput analysis of gene expression" NATURE GENETICS, NATURE AMERICA, NEW YORK, US, vol. 28, no. 1, May 2001 (2001-05), pages 21-28, XP002266527 ISSN: 1061-4036 * abstract * * page 23, right-hand column, paragraph 2 - page 28, left-hand column, last paragraph * | 1-20 | TECHNICAL FIELDS SEARCHED (Int.Cl.7)<br><br>G06F |
| | -/-- | | |

The present search report has been drawn up for all claims

| Place of search | Date of completion of the search | Examiner |
|---|---|---|
| Munich | 26 April 2005 | Hilbig, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 691 311 A1**

European Patent
Office

**EUROPEAN SEARCH REPORT**

Application Number

EP 05 29 0333

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HARTEMINK A J ET AL: "Bayesian methods for elucidating genetic regulatory networks" IEEE INTELLIGENT SYSTEMS IEEE USA, vol. 17, no. 2, March 2002 (2002-03), pages 37-43, XP002325973 ISSN: 1094-7167 * page 37, right-hand column, paragraph 2 - page 38, right-hand column, paragraph 2 * * figure 1 * | 1-20 | |
| A | DATTA SUSMITA ET AL: "Comparisons and validation of statistical clustering techniques for microarray gene expression data." BIOINFORMATICS (OXFORD), vol. 19, no. 4, 1 March 2003 (2003-03-01), pages 459-466, XP002325974 ISSN: 1367-4803 * abstract * * page 460, right-hand column, last paragraph - page 462, left-hand column, paragraph 1 * * page 464, right-hand column, paragraph 4 - page 466, left-hand column, last paragraph * | 1-20 | TECHNICAL FIELDS SEARCHED (Int.Cl.7) |

-/--

The present search report has been drawn up for all claims

| Place of search | Date of completion of the search | Examiner |
|---|---|---|
| Munich | 26 April 2005 | Hilbig, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

23

**European Patent Office**

**EUROPEAN SEARCH REPORT**

Application Number

EP 05 29 0333

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | SLONIM DONNA K: "From patterns to pathways: Gene expression data analysis comes of age." NATURE GENETICS, vol. 32, no. Supplement, December 2002 (2002-12), pages 502-508, XP002325975 ISSN: 1061-4036 * abstract * * page 502, left-hand column, paragraph 3 - page 507, right-hand column, last paragraph * ----- | 1-20 | |
| A | NADON R ET AL: "Statistical issues with microarrays: processing and analysis" TRENDS IN GENETICS, ELSEVIER, AMSTERDAM, NL, vol. 18, no. 5, 1 May 2002 (2002-05-01), pages 265-271, XP004359977 ISSN: 0168-9525 * abstract * * page 265, left-hand column, paragraph 1 - page 266, left-hand column, last paragraph * * page 269, left-hand column, paragraph 1 - page 271, right-hand column, last paragraph * * figure 1 * ----- | 1-20 | TECHNICAL FIELDS SEARCHED (Int.Cl.7) |

The present search report has been drawn up for all claims

| Place of search | Date of completion of the search | Examiner |
|---|---|---|
| Munich | 26 April 2005 | Hilbig, M |

EPO FORM 1503 03.82 (P04C01)